# EUROPEAN PATENT APPLICATION

(11) **EP 2 647 294 A1**
(43) Date of publication of application: **09.10.2013**
(21) Application number: 11821850.2
(22) Date of filing: 31.08.2011
(51) Int. Cl.: A23C 9/127, C12N 1/20, C12N 15/09, C12R 1/01

(54) **METHOD FOR PRODUCING FERMENTED FOOD CONTAINING BIFIDOBACTERIUM BACTERIA**

(30) Priority: 31.08.2010 JP 2010194521
(71) Applicant: Morinaga Milk Industry Co., Ltd., Minato-ku Tokyo 108-8384 (JP)
(72) Inventor: ODAMAKI, Toshitaka, Zama-shi Kanagawa 252-8583 (JP); YONEZAWA, Sumiko, Zama-shi Kanagawa 252-8583 (JP); MARUYAMA, Hiroshi, Zama-shi Kanagawa 252-8583 (JP); TAKAHASHI, Noritoshi, Zama-shi Kanagawa 252-8583 (JP)
(74) Representative: Dean, John Paul
(86) International application number: PCT/JP2011/069726
(87) International publication number: WO 2012/029835

(57) **Abstract**

A milk raw material is fermented by using *Lactococcus lactis* not having cell wall-enveloped proteinase and a *Bifidobacterium* bacterium. The *Lactococcus lactis* is, for example, a *Lactococcus lactis* having a property that, when this bacterium and the *Bifidobacterium* bacterium are inoculated into a medium containing 1% (W/W) of glucose and 10% (W/W) of reduced skim milk powder in amounts of 5.0 x 10⁶ to 2.0 x 10⁸ CFU and 1.0 x 10⁷ to 3.0 x 10⁹ CFU per 1 ml of the medium, respectively, and cultured, and the medium is rapidly cooled to 10°C from culture temperature when pH of the medium becomes 4.6 to 5.5, and stored at 10°C for 2 weeks, survival rate of the *Bifidobacterium* bacterium is maintained to be 30% or more.

SEQUENCE LISTING

<110>  Morinaga Milk Industry Co., Ltd.

<120>  A method for producing a fermented food containing Bifidobacteria

<130>  FP218-11174

<150>  JP2010-194521
<151>  2010-08-31

<160>  4

<170>  PatentIn version 3.5


<210>  1
<211>  22
<212>  DNA
<213>  Artificial sequence

<220>
<223>  primer GBf

<400>  1
gcaaatacgg tgacggctgc ga                                                22


<210>  2
<211>  25
<212>  DNA
<213>  Artificial sequence

<220>
<223>  primer GB2r

<400>  2
tgagcattat aataggtctt cttcc                                             25


<210>  3
<211>  22
<212>  DNA
<213>  Artificial sequence

<220>
<223>  primer GHf

<400>  3
caaatacggt gacggctgct aa                                                22


<210>  4
<211>  24
<212>  DNA
<213>  Artificial sequence

<220>
<223>  primer GH2r

<400>  4
tagcattata ataggtcttc gtca                                              24

## Description

### Technical Field

The present invention relates to a method for producing a fermented food obtainable by fermentation of a milk raw material using *Lactococcus lactis* and a *Bifidobacterium* bacterium, and *Lactococcus lactis* suitably used for the method.

### Background Art

*Bifidobacterium* bacteria (hereafter also referred to as "bifidobacteria") such as *Bifidobacterium longum* constitute one class of dominant species of intestinal flora formed in the human intestinal tract. It is known that bifidobacteria have intestinal function-controlling action for restoring balance of enterobacteria, immunity-enhancing action, carcinogenesis-suppressing action, and so forth. Therefore, demands for foods containing live bifidobacteria, such as bifidobacterium-fermented milk, are increasing in recent years with the rise of health consciousness of consumers.

Bifidobacteria show poor proliferation in a lactic medium. Therefore, for example, in order to obtain a bifidobacterium content of 1 x 10⁷ CFU (colony forming unit)/mL, a certain amount of various growth promoting substances are usually added to fermented milk.

However, the growth promoting substances are generally expensive, and may also degrade flavors of foods. Moreover, it is difficult to store bifidobacteria under acidic conditions, and they are easily killed under such conditions. For this reason, even if bifidobacteria once proliferate during manufacture of a fermented dairy product or the like, amount of live bifidobacteria in the fermented dairy product or the like decreases at an accelerating pace during distribution of the fermented dairy product or the like.

Therefore, a current theme of research is to improve viability and storage survivability of bifidobacteria and thereby produce a fermented milk containing a large amount of live bifidobacteria, especially, a fermented milk abundantly containing live bifidobacteria even at the time of ingestion by consumers at a level similar to that observed immediately after the manufacture thereof.

There have been disclosed various methods for improving viability and storage survivability of bifidobacteria by performing mixed fermentation using a bifidobacterium and another lactic acid bacterium without adding such growth promoting substances as described above or the like.

Concerning the method for improving viability of bifidobacteria in the manufacture of fermented milk, there have been disclosed, for example, yogurt containing *Lactococcus lactis* subsp. *lactis, Lactococcus lactis* subsp. *cremoris,* and a bifidobacterium, and a method for producing it (refer to, for example, Patent document 1).

In addition, concerning the method for improving storage survivability of bifidobacteria in fermented milk, there has been disclosed, for example, a method for producing bifidobacterium-fermented milk comprising mixed culture of *Bifidobacterium breve* and *Lactococcus lactis* subsp. *lactis* not producing diacetyl and acetoin in a medium mainly consisting of milk (refer to, for example, Patent document 2).

Moreover, there have been disclosed, for example, a method for producing fermented milk comprising fermentation of a fermentation base using *Lactococcus lactis* having a cell wall-enveloped proteinase (PrtP) and a *Bifidobacterium* bacterium (refer to, for example, Patent document 3), a method for producing a composition containing *Bifidobacterium* bacteria comprising inoculating a *Bifidobacterium* bacterium into a medium containing milk proteins to which disrupted cells of a lactic acid bacterium having a cell wall-enveloped proteinase or a fraction of the enzyme fractionated from the cells of the lactic acid bacterium was added (refer to, for example, Patent document 4), a method for producing fermented milk characterized by using a *Lactococcus* bacterium having fermentation ability in a 10% reduced skim milk powder medium, and having a proliferation-promoting effect and survivability-improving effect on *Bifidobacterium longum,* and fermented milk produced by this production method (refer to, for example, Patent document 5).

### Prior art references

### Patent documents

Patent document 1: Japanese Patent No. 3364491
Patent document 2: Japanese Patent No. 3068484
Patent document 3: Japanese Patent No. 4448896
Patent document 4: Japanese Patent Laid-open (KOKAI) No. 2009-296910
Patent document 5: International Patent Publication WO2008/099543

### Summary of the Invention

### Object to be Achieved by the Invention

Although various methods for improving viability and storage survivability of bifidobacteria have been disclosed as described above, they still leave room for further improvement. For example, in the method of Patent document 1 mentioned above, growth of bifidobacteria is promoted so that fermentation time can be shortened, but Patent document 1 does not refer to storage survivability of the bifidobacteria at all.

In the method of Patent document 2 mentioned above, the use of mixed bacteria consisting of a specific bifidobacterium and a specific lactic acid bacterium provides both proliferation-promoting effect and survivability-improving effect, but Patent document 2 does not refer at all to bifidobacteria other than *Bifidobacterium breve,* for example, *Bifidobacterium longum,* which is currently widely used for foodstuffs. In fact, in an experiment using the strain mentioned in Patent document 2 (FERM BP-6224), sufficient survivability of *Bifidobacterium longum* could not be obtained, as described herein later.

Further, in the method described in Patent document 3 mentioned above, use of the *Lactococcus lactis* having a cell wall-enveloped proteinase (PrtP) provides an effect of promoting proliferation of bifidobacteria, but PrtP produces many oligopeptides and amino acids from proteins to generate bitterness and umami taste, and therefore flavor of fermented milk may be affected (for example, Pillidge, C.J. et al., Int. Dairy Journal, 2003, 13:345-354). The method described in Patent document 4 has the same problem.

Furthermore, according to Yonezawa S. et al., J. Dairy Sci., 2010, 93:1815-23 and Patent document 3, *Lactococcus lactis* having fermentation ability in a skim milk powder medium had PrtP, and therefore the method of Patent document 5 also has the same problem as that of Patent document 3, namely, PrtP may affect on flavor of fermented milk.

On the other hand, since *Lactococcus lactis* not having PrtP is not able to incorporate sufficient nutrition from a skim milk powder medium, substantially all the strains thereof could not grow in a skim milk powder medium, and proliferation-promoting effect and survivability-improving effect thereof on bifidobacteria have not been observed.

An object of the present invention is to provide a method for producing a fermented food having good flavor by using a lactic acid bacterium that can improve storage survivability of *Bifidobacterium* bacteria, a fermented food produced by such a production method, and a starter for fermentation of a milk raw material containing a *Bifidobacterium* bacterium and the above-mentioned lactic acid bacterium.

### Means for Achieving the Object

The inventors of the present invention conducted various researches in order to achieve the aforementioned object, as a result, found that there existed a strain of *Lactococcus lactis* not having cell wall-enveloped proteinase PrtP that improved storage property of a *Bifidobacterium* bacterium in mixed fermentation of the strain and the *Bifidobacterium* bacterium, and thus accomplished the present invention.

The present invention thus relates to a method for producing a fermented food comprising fermenting a milk raw material by using *Lactococcus lactis* not having cell wall-enveloped proteinase and a *Bifidobacterium* bacterium.

The present invention also provides a fermented food produced by the method.

The present invention also provides a starter for fermentation of a milk raw material containing a *Bifidobacterium* bacterium, which comprises *Lactococcus lactis* not having cell wall-enveloped proteinase.

In preferred embodiments of the method for producing a fermented food and the starter for fermentation of a milk raw material containing a *Bifidobacterium* bacterium of the present invention, the *Lactococcus lactis* has a property that, when this bacterium is inoculated into a medium containing 1% (W/W) of glucose and 10% (W/W) of reduced skim milk powder in an amount of 5.0 x 10⁶ to 2.0 x 10⁸ CFU per 1 ml of the medium, and cultured at 37°C for 4 to 24 hours, the medium is not coagulated.

In a preferred embodiment of the present invention, the *Lactococcus lactis* has a property that, when this bacterium and the *Bifidobacterium* bacterium are inoculated into a medium containing 1% (W/W) of glucose and 10% (W/W) of reduced skim milk powder in amounts of 5.0 x 10⁶ to 2.0 x 10⁸ CFU and 1.0 x 10⁷ to 3.0 x 10⁹ CFU per 1 ml of the medium, respectively, and cultured, and the medium is rapidly cooled to 10°C from culture temperature when pH of the medium becomes 4.6 to 5.5, and stored at 10°C for 2 weeks, dissolved oxygen concentration in the medium is maintained to be 2 ppm or lower.

In a preferred embodiment of the present invention, the *Lactococcus lactis* has a property that, when this bacterium and the *Bifidobacterium* bacterium are inoculated into a medium containing 1% (W/W) of glucose and 10% (W/W) of reduced skim milk powder in amounts of 5.0 x 10⁶ to 2.0 x 10⁸ CFU and 1.0 x 10⁷ to 3.0 x 10⁹ CFU per 1 ml of the medium, respectively, and cultured, and the medium is rapidly cooled to 10°C from culture temperature when pH of the medium becomes 4.6 to 5.5, and stored at 10°C for 2 weeks, survival rate of the *Bifidobacterium* bacterium is maintained to be 30% or more.

In a preferred embodiment of the present invention, the *Lactococcus lactis* has a property that, when this bacterium, the *Bifidobacterium* bacterium, *Streptococcus thermophilus* and *Lactobacillus delbrueckii* subsp. *bulgaricus* are inoculated into a medium containing 1% (W/W) of glucose and 10% (W/W) of reduced skim milk powder in amounts of 5.0 x 10⁶ to 2.0 x 10⁸ CFU, 1.0 x 10⁷ to 3.0 x 10⁹ CFU, 2.0 x 10⁵ to 9.0 x 10⁷ CFU and 2.0 x 10⁵ to 9.0 x 10⁷ CFU per 1 ml of the medium, respectively, and cultured at 37°C for 3 to 24 hours, the medium is coagulated.

In a preferred embodiment of the present invention, the *Lactococcus lactis* is selected from the group consisting of *Lactococcus lactis* subsp. *lactis* and *Lactococcus lactis* subsp. *cremoris.*

In a preferred embodiment of the present invention, the *Lactococcus lactis* is selected from the group consisting of *Lactococcus lactis* subsp. *lactis* LcL13 (FERM BP-11276), *Lactococcus lactis* subsp. *lactis* LcL26 (FERM BP-11277), and *Lactococcus lactis* subsp. *cremoris* LcC46 (FERM BP-11275).

In a preferred embodiment of the present invention, the *Bifidobacterium* bacterium is *Bifidobacterium longum.*

In a preferred embodiment of the present invention, *Bifidobacterium longum* is the *Bifidobacterium longum* ATCC BAA-999 strain.

In a preferred embodiment of the present invention, a lactic acid bacterium selected from the group consisting of *Streptococcus thermophilus* and *Lactobacillus delbrueckii* is used for the fermentation.

The present invention also provides a bacterial strain selected from the group consisting of *Lactococcus lactis* subsp. *lactis* LcL13 (FERM BP-11276), *Lactococcus lactis* subsp. *lactis* LcL26 (FERM BP-11277), and *Lactococcus lactis* subsp. *cremoris* LcC46 (FERM BP-11275).

### Brief Description of the Drawing

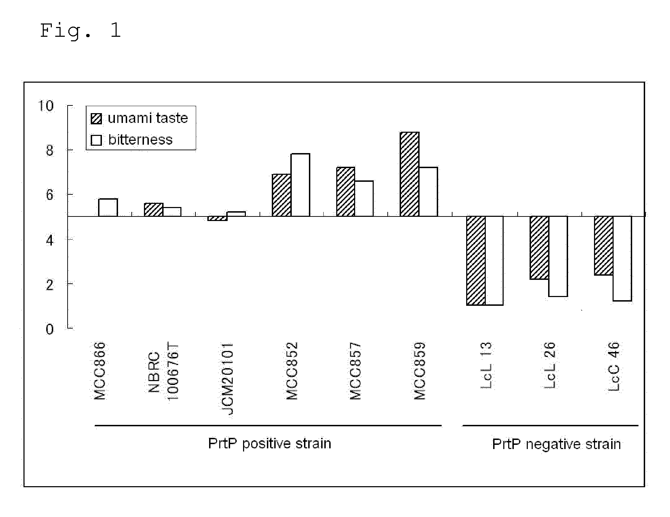

Fig. 1 shows results of evaluation for taste of yogurt produced by the method of the present invention.

### Embodiments for Carrying out the Invention

Hereafter, the present invention will be explained in detail.

The bacterium used for the present invention is *Lactococcus lactis* not having cell wall-enveloped proteinase. The cell wall-enveloped proteinase (EC 3.4.21.96, also referred to as "PrtP") is an enzyme which is present in the cell membrane, of which active site is exposed on the cell surface. As the PrtP enzyme of *Lactococcus lactis,* there are known those of PI type (the enzyme of this type scarcely decomposes α-casein, but well decomposes β-casein from the neighborhood of the C-terminus), PIII type (the enzyme of this type well decomposes both α-casein and β-casein from both the C-terminus and N-terminus), and an intermediate type of them (PI/PIII type) (for example, Reid, J.R. et al., Applied and Environmental Microbiology, 1994, Vol. 60, No. 3, pp.801-806). Specific examples of PrtP include, as PrtP of *Lactococcus lactis,* PrtP and homologues thereof, of which gene sequences are registered at NCBI with accession numbers of AY542690, AY542691 etc. Examples of the homologues include proteins having an amino acid sequence encoded by any of the aforementioned gene sequences, but including substitutions, deletions, insertions or additions of one or several amino acid residues, and having the PrtP activity. The number meant by the expression of one or several is preferably 1 to 20, more preferably 1 to 10, still more preferably 1 to 5, particularly preferably 1 to 3. Examples of the homologues further include proteins showing a homology of 80% or more, preferably 90% or more, more preferably 95% or more, to an amino acid sequence encoded by the nucleotide sequence of the accession number AY542690 or AY542691, and having the PrtP activity.

The expression of bacterium "not having PrtP" means that the bacterium does not have the enzymatic activity of PrtP, and this state include a state that the bacterium does not have any PrtP protein, and a state that the bacterium has a PrtP protein, but the PrtP protein does not have the enzymatic activity. Moreover, a state that the bacterium has a PrtP protein, but amount or activity of the PrtP protein is markedly lower than that of a bacterium having PrtP is also included in the state of "not having PrtP".

Although subspecies (subsp.) of the *Lactococcus lactis* is not particularly limited, a subspecies including a strain having PrtP and a strain not having PrtP is preferred. Specific examples include, for example, *Lactococcus lactis* subsp. *lactis* and *Lactococcus lactis* subsp. *cremoris.* A *Lactococcus lactis* strain which does not have PrtP can be obtained by selecting a strain not having the PrtP activity, or a strain not having a gene coding for PrtP from the nature.

Since PrtP has an enzymatic active site outside the cell, a strain having PrtP can decompose proteins in the medium, and use them for growth of the strain itself. However, a strain not having PrtP cannot use proteins in the medium for growth of the strain. Therefore, a strain not having PrtP can be selected on the basis of observation of growth in a medium containing proteins, for example, a medium containing 10% (W/W) of reduced skim milk powder.

Further, a strain not having a gene coding for PrtP can be selected on the basis of detection of a PrtP gene or a part thereof by PCR, for example, as described in the examples. Examples of primers for amplifying a PrtP gene include a set of primers of SEQ ID NOS: 1 and 2 and a set of primers of SEQ ID NOS: 3 and 4.

A strain not having PrtP can also be obtained from a strain having PrtP by inactivating, disrupting or deleting a PrtP gene using mutagenesis or gene recombination. PrtP encoded by a PrtP gene to be inactivated, disrupted or deleted may be the enzyme of PI type, PIII type or PI/PIII type.

One embodiment of the *Lactococcus lactis* not having PrtP is a strain having a property that, when this bacterium is inoculated into a medium containing 1% (W/W) of glucose and 10% (W/W) of reduced skim milk powder in an amount of 5.0 x 10⁶ to 2.0 x 10⁸ CFU per 1 ml of the medium, and cultured at 37°C for 4 to 24 hours, for example, 16 hours, the medium is not coagulated.

The medium containing 1% (W/W) of glucose and 10% (W/W) of reduced skim milk powder can be prepared by dissolving glucose at 1% (W/W) and reduced skim milk powder at 10% (W/W) in water, and sterilizing the solution. The sterilization can be performed by, for example, a heat treatment at 80 to 122°C for 40 to 5 minutes, preferably at 85 to 95°C for 35 to 5 minutes.

The cell count (CFU) of the bacterium can be measured by spreading an appropriately diluted suspension of the bacterium on an appropriate agar medium, for example, a BCP-added plate count agar medium (produced by EIKEN CHEMICAL CO., LTD.) to perform culture, and counting colonies that appear.

Whether the medium is coagulated or not can be determined by, for example, performing culture using a test tube or the like. Specifically, when a test tube containing the medium is inverted, if the medium does not show fluidity, it is judged that the medium is coagulated, and if the medium shows fluidity, it is judged that the medium is not coagulated.

Further, the *Lactococcus lactis* not having PrtP preferably has a property that when, into a medium containing 1% (W/W) of glucose and 10% (W/W) of reduced skim milk powder, the bacterium is inoculated in an amount of 5.0 x 10⁶ to 2.0 x 10⁸ CFU per 1 ml of the medium, the *Bifidobacterium* bacterium is inoculated in an amount of 1.0 x 10⁷ to 3.0 x 10⁹ CFU, preferably 1.0 x 10⁷ to 5.0 x 10⁷ CFU, per 1 ml of the medium, they are cultured, and the medium is rapidly cooled to 10°C from culture temperature when pH of the medium becomes 4.6 to 5.5 and stored at 10°C for 2 weeks, dissolved oxygen concentration in the medium is maintained to be 2 ppm or lower, preferably 1 ppm or lower, more preferably 0.5 ppm or lower. If dissolved oxygen concentration in the medium is high, *Bifidobacterium* bacteria hardly grow. Therefore, when a milk raw material is fermented by using *Lactococcus lactis* together with a *Bifidobacterium* bacterium, the *Lactococcus lactis* is preferably *Lactococcus lactis* that does not increase dissolved oxygen concentration in the medium.

The rapid cooling to 10°C is desirably performed preferably within 1 hour, more preferably within 30 minutes, particularly preferably within 10 minutes. The culture temperature is preferably 30 to 40°C, more preferably 36 to 38°C, particularly preferably 37°C. The same shall apply to the following descriptions.

Further, in another embodiment of the present invention, the *Lactococcus lactis* not having PrtP preferably has a property that when, into a medium containing 1% (W/W) of glucose and 10% (W/W) of reduced skim milk powder, the bacterium is inoculated in an amount of 5.0 x 10⁶ to 2.0 x 10⁸ CFU per 1 ml of the medium, the *Bifidobacterium* bacterium is inoculated in an amount of 1.0 x 10⁷ to 3.0 x 10⁹ CFU, preferably 1.0 x 10⁷ to 5.0 x 10⁷ CFU, per 1 ml of the medium, they are cultured, and the medium is rapidly cooled to 10°C from culture temperature when pH of the medium becomes 4.6 to 5.5 and stored at 10°C for 2 weeks, survival rate of the *Bifidobacterium* bacterium is maintained to be 30% or more, preferably 50% or more, more preferably 80% or more. The survival rate refers to a rate of live cell count after the storage to live cell count at the time of the start of the storage. Although this property and the aforementioned property of not increasing dissolved oxygen concentration in the medium may be independently possessed by the *Lactococcus lactis,* the *Lactococcus lactis* preferably have both properties.

Further, in another embodiment of the present invention, the *Lactococcus lactis* not having PrtP preferably has a property that when, into a medium containing 1% (W/W) of glucose and 10% (W/W) of reduced skim milk powder, this bacterium is inoculated in an amount of 5.0 x 10⁶ to 2.0 x 10⁸ CFU per 1 ml of the medium, the *Bifidobacterium* bacterium is inoculated in an amount of 1.0 x 10⁷ to 3.0 x 10⁹ CFU, preferably 1.0 x 10⁷ to 5.0 x 10⁷ CFU, per 1 ml of the medium, *Streptococcus thermophilus* and *Lactobacillus delbrueckii* subsp. *bulgaricus* are inoculated each in an amount of 2.0 x 10⁵ to 9.0 x 10⁷ CFU per 1 ml of the medium, and they are cultured at 37°C for 4 to 24 hours, for example, 8 hours, the medium is coagulated.

Although a fact that the *Lactococcus lactis* not having PrtP does not coagulate a medium containing reduced skim milk powder means that the bacterium does not have fermentation ability by itself in the medium, a fact that when the bacterium is cultured with a *Bifidobacterium* bacterium in the medium, dissolved oxygen concentration in the medium is reduced, and the medium is coagulated, namely, pH of the medium is reduced, as well as the survival rate of the *Bifidobacterium* bacterium after fermentation is increased means that the *Lactococcus lactis* of the present invention is suitable for production of a fermented food containing a *Bifidobacterium* bacterium from a milk raw material. Therefore, *Lactococcus lactis* not having PrtP, especially a strain thereof having the aforementioned properties, is suitable as a starter for producing a fermented food containing a *Bifidobacterium* bacterium. The "starter for fermentation of a milk raw material containing a *Bifidobacterium* bacterium" of the present invention refers to a starter for producing such a fermented food containing a *Bifidobacterium* bacterium, i.e., a bacterium to be inoculated into a milk raw material together with a *Bifidobacterium* bacterium in order to produce a fermented food.

Specific examples of the *Lactococcus lactis* used for the present invention include *Lactococcus lactis* subsp. *lactis* LcL13 (FERM BP-11276), *Lactococcus lactis* subsp. *lactis* LcL26 (FERM BP-11277) and *Lactococcus lactis* subsp. *cremoris* LcC46 (FERM BP-11275).

These strains were deposited at the independent administrative institution, National Institute of Advanced Industrial Science and Technology, International Patent Organism Depositary (Tsukuba Central 6, 1-1, Higashi 1-Chome, Tsukuba-shi, Ibaraki-ken, 305-8566, Japan) on August 11, 2010 as international deposits under the provisions of the Budapest Treaty with the accession numbers mentioned in the parentheses, respectively.

The method of the present invention is a method for producing a fermented food comprising fermenting a milk raw material using *Lactococcus lactis* not having PrtP and a *Bifidobacterium* bacterium.

The *Bifidobacterium* bacterium is not particularly limited, and examples include *Bifidobacterium longum, Bifidobacterium bifidum, Bifidobacterium breve, Bifidobacterium adolescentis,* and *Bifidobacterium infantis* (this species is reclassified as *Bifidobacterium longum* subsp. *infantis).* Among these, *Bifidobacterium longum* is preferred. Specific examples of *Bifidobacterium longum* include the *Bifidobacterium longum* ATCC BAA-999 strain. This strain can be purchased from, for example, American Type Culture Collection (Address: 12301 Parklawn Drive, Rockville, Maryland 20852, United States of America).

The milk raw material is not particularly limited, so long as there is chosen a material derived from milk from which a fermented food can be produced by fermentation using *Lactococcus lactis* not having PrtP and a *Bifidobacterium* bacterium, as well as another lactic acid as required. Examples include, for example, milk and fractionation products and processed products thereof, such as cow's milk, skim milk, fresh cream, butter, dry whole milk, skim milk powder, and those obtained by mixing, dissolving or suspending the foregoing materials with or in water. The milk raw material may contain sweetener such as sucrose, pectin, fruits, fruit juice, agar, gelatin, fat or oil, flavor, coloring agent, stabilizer, reducing agent, and so forth, as required. The milk raw material may be subjected to sterilization, homogenization, cooling, or the like in a conventional manner before use in the fermentation.

Although amounts of the *Lactococcus lactis* not having PrtP and the *Bifidobacterium* bacterium to be inoculated into the milk raw material are not particularly limited, the *Lactococcus lactis* is preferably inoculated in an amount of 10⁴ to 10⁸ CFU/ml of the milk raw material, more preferably in an amount of 10⁶ to 10⁷ CFU/ml of the milk raw material, and the *Bifidobacterium* bacterium is preferably inoculated in an amount of 10⁵ to 10⁹ CFU/ml of the milk raw material, more preferably in an amount of 10⁷ to 10⁸ CFU/ml of the milk raw material. Further, although ratio of the inoculation amounts of the *Lactococcus lactis* not having PrtP to the *Bifidobacterium* bacterium (ratio of bacterial counts) is not also particularly limited, it is preferably 1000:1 to 1:10, more preferably 10:1 to 1:1.

The *Lactococcus lactis* not having PrtP to be inoculated into the milk raw material may consist of a single kind of strain, or two or more kinds of strains. The *Bifidobacterium* bacterium may also consist of a single kind of strain, or two or more kinds of strains.

The *Lactococcus lactis* and the *Bifidobacterium* bacterium, as well as another lactic acid bacterium used as required, which are to be inoculated into the milk raw material, are preferably cultured beforehand in another medium as seed culture or preculture. Although the medium is not particularly limited so long as there is used a medium suitable for culture of the *Lactococcus lactis* and the *Bifidobacterium* bacterium, as well as another lactic acid bacterium used as required, examples include, for example, a medium containing reduced skim milk powder. Concentration of the reduced skim milk powder is preferably 3% (W/W) or higher, particularly preferably 8% (W/W) or higher. The medium used for the seed culture or preculture may contain growth-promoting substances such as yeast extract, reducing agents such as L-cysteine, and so forth. In particular, since *Bifidobacterium* bacteria show poor proliferation in a medium containing reduced skim milk powder, it is preferable to use a medium containing a growth-promoting substance, for example, 0.1 to 1% (W/W) of yeast extract. The conditions for sterilization of the medium are the same as those mentioned above.

The fermentation may be performed by adding another lactic acid bacterium to the milk raw material as required, in addition to the *Lactococcus lactis* not having PrtP and the *Bifidobacterium* bacterium. The other lactic acid bacterium is not particularly limited so long as there is used a lactic acid bacterium that can be used for manufacture of foodstuffs and does not inhibit growth of the *Lactococcus lactis* and the *Bifidobacterium* bacterium. Examples include, for example, *Streptococcus thermophilus, Lactobacillus* delbrueckii, and so forth, for the case that where the fermented food is yogurt. The lactic acid bacterium may consist of a single kind of strain, or may consist of two or more kinds of strains.

When milk is fermented by using the *Lactococcus lactis* not having PrtP and the *Bifidobacterium* bacterium, pH of the culture is usually around 5, and drinkable yogurt is usually obtained. If the fermentation is performed by further using a lactic acid bacterium such as *Streptococcus thermophilus* or *Lactobacillus delbrueckii,* pH is reduced, and yogurt having a stronger structure (yogurt that can be taken in with a spoon) can be produced.

Although ratio (ratio of bacterial counts) of the inoculation amount of the *Lactococcus lactis* and the *Bifidobacterium* bacterium to the inoculation amount of the other lactic acid bacterium is not particularly limited, it is preferably 1000:1 to 10:1.

The order of inoculations of the *Lactococcus lactis,* the *Bifidobacterium* bacterium and the other lactic acid bacterium into the milk raw material is not particularly limited, and they may be simultaneously inoculated. Further, arbitrary bacterium or bacteria among these bacteria may be inoculated two or more times.

The fermentation conditions such as culture temperature and culture time may be the same as those used for usual manufacture of fermented foods from a milk raw material. For example, the culture temperature is preferably 30 to 40°C, more preferably 36 to 38°C. The culture time can be suitably determined according to type of fermented food to be produced, and it is usually preferably 3 to 18 hours.

The obtained fermented food can be appropriately processed like usual fermented foods obtained from a milk raw material. For example, the fermented food as it is after the fermentation may be used as a foodstuff, or it may be homogenized and liquefied. Furthermore, sweetener such as sucrose, pectin, fruits, fruit juice, agar, gelatin, fat or oil, perfume, coloring agent, stabilizer, reducing agent, and so forth may be added. The fermented food may be filled in a container as required.

The fermented food produced as described above shows less bitterness and umami taste resulting from decomposition of proteins, and it shows superior storage survivability of *Bifidobacterium* bacteria.

### Examples

Hereafter, the present invention will be still more specifically explained with reference to test examples and examples.

### Test Example 1: Acquisition of Lactococcus lactis strains

In order to obtain a strain of *Lactococcus lactis* not having PrtP enzyme from the nature, samples collected from the nature in Japan were diluted with a diluent for anaerobic bacteria (Mitsuoka T., "World of Enteric Bacteria", Soubunsha, p.322, 1980), the diluted samples were applied to plates of Briggs liver broth having the following composition (containing 15 g/L of agar, ibid., p.319), and culture was performed at 30°C under an anaerobic condition.

### [Diluent for anaerobic bacteria]

| | |
|---|---|
| Salt solution I (0.78% K₂HPO₄ solution) | 37.5 ml |
| Salt solution II (solution containing 0.47% of KH₂PO₄, 1.18% of NaCl, 1.20% of (NH₄)₂SO₄, 0.12% of CaCl₂, and 0.25% of MgSO₄·H₂O) | 37.5 ml |
| Resazurin (0.1% aqueous solution) | 1 ml |
| L-Cysteine HCl·H₂O | 0.5 g |
| L-Ascorbic acid (25% aqueous solution) | 2 ml |
| Na₂CO₃ (8% solution) | 50 ml |
| Agar | 0.5 g |
| Purified water | 860 ml |

### [Briggs liver broth]

| | |
|---|---|
| Tomato juice exudate | 400 ml |
| Neopeptone (Difco) | 15 g |
| Yeast extract (Difco) | 6 g |
| Liver extract | 75 ml |
| Glucose | 20 g |
| Soluble starch | 0.5 g |
| NaCl | 5 g |
| Tween 80 | 1 g |
| L-Cysteine HCl·H₂O | 0.2 g |
| Purified water | 525 ml |
| pH 6.8 | |

Then, bacteria showing morphology of streptococci and determined as Gram positive on the basis of microscopic observation of smear were picked up from the obtained colonies. These bacteria were streaked on the BL agar medium having the following composition, and anaerobic culture was repeated in the same manner as that described above to obtain purely isolated strains.

### [BL agar medium]

| | |
|---|---|
| Lab-Lemco Powder (Oxoid) | 2.4 g |
| Proteose peptone No. 3 (Difco) | 10 g |
| Trypticase (BBL) | 5 g |
| Phytone | 3 g |
| Yeast extract (Difco) | 5 g |
| Liver extract | 150 ml |
| Glucose | 10 g |
| Soluble starch | 0.5 g |
| Solution A | 10 ml |
| Toray silicone SH5535 (10% solution) | 5 ml |
| Tween 80 | 1 g |
| Agar | 15 g |
| L-Cysteine HCl·H₂O | 0.5 g |
| Horse blood | 50 ml |
| pH 7.2 | |

| | |
|---|---|
| Liver extract: Liver powder (10 g, Kyokuto) was extracted with 170 ml of purified water on a warmed water bath at 50 to 60°C for about 1 hour, and then the extract was heated at 100°C for several minutes, adjusted to pH 7.2, and filtered through filter paper. Solution A: 15 g of MgSO₄·7H₂O, 0.5 g of FeSO₄·7H₂O_{,} 0.5 g of NaCl, and 0.337 g of MnSO₄ were dissolved in 250 ml of purified water. | |

The ingredients other than L-cysteine and the horse blood are dissolved on a water bath, pH of the solution are adjusted, the solution is subjected to sterilization at 115°C for 20 minutes and then cooled to 50°C, L-cysteine and horse blood are added to the solution, and the mixture are poured into petri dishes to obtain the plate medium.

The nucleotide sequences of the genomic DNAs of these strains were determined in a conventional manner. Homology search was performed for the full length of the 16S ribosomal RNA gene sequence on the international nucleotide sequence database (GenBank) of NCBI (National Center for Biotechnology Information) by using BLAST (Basic Local Alignment Search Tool, http://blast.ncbi.nlm.nih.gov/Blast.cgi), and 280 strains of *Lactococcus* bacteria showing a homology of 98% or higher for each type strain were identified, as a result. Among 242 strains showing a homology of 98% or higher with respect to *Lactococcus lactis,* strains of the group assimilating lactose and showing the highest homology to *Lactococcus lactis* subsp. *lactis* among the type strains of *Lactococcus lactis* subspecies were identified as *Lactococcus lactis* subsp. *lactis*. Further, strains of the group showing the highest homology to *Lactococcus lactis* subsp. *cremoris* among the type strains of *Lactococcus lactis* subspecies were identified as *Lactococcus lactis* subsp. *cremoris.* All the obtained strains were non-sporulating and non-motile facultative anaerobic Grampositive cocci, and were negative for both catalase and gas production.

Then, it was confirmed whether the obtained *Lactococcus lactis* strains had a PrtP enzyme or not. Specifically, colony of each strain on the BL agar medium was inoculated into the Difco (registered trademark) M17 Broth (produced by Becton, Dickinson and Company) containing 0.5% each of lactose and glucose with a platinum loop, and cultured at 30°C for 16 hours. The obtained culture broth was inoculated into the same medium at a concentration of 3%, and culture was performed at 30°C for 16 hours. The cells were obtained by centrifugation, DNA was extracted by using DNeasy Blood and Tissue Kit (produced by QIAGEN K.K.), and whether the PrtP gene was contained in DNA was confirmed by PCR.

PCR was performed according to the method described in Journal of Applied Microbiology, 2006, vol. 100, pp.1307-1317. As the primers, a primer set of a forward primer GBf (GCAAATACGGTGACGGCTGCGA, SEQ ID NO: 1) and a reverse primer GB2r (TGAGCATTATAATAGGTCTTCTTCC, SEQ ID NO: 2), or a primer set of a forward primer GHf (CAAATACGGTGACGGCTGCTAA, SEQ ID NO: 3) and a reverse primer GH2r (TAGCATTATAATAGGTCTTCGTCA, SEQ ID NO: 4) was used. As a result, it was confirmed that 128 strains among the 242 strains of *Lactococcus lactis* had the PrtP gene. On the other hand, it was found that the remaining 114 strains did not have the PrtP gene.

### Test Example 2: Fermentation ability test for Lactococcus lactis strains in lactic medium

Culture broth of each of the strains obtained in Test Example 1 and strains mentioned in Table 1 was inoculated at a concentration of 3% into the Difco (registered trademark) M17 Broth (produced by Becton, Dickinson and Company) containing 0.5% each of lactose and glucose, and culture was performed at 30°C for 16 hours. The cells were collected by centrifugation, washed, and then suspended in a lactic medium (1% (W/W) of glucose, 10% (W/W) of reduced skim milk powder (produced by Morinaga Milk Industry Co., Ltd.)) of the same volume as that of the original culture medium to obtain seed culture. The NBRC12007 and NBRC100676 strains can be obtained from the independent administrative institution, National Institute of Technology and Evaluation (2-5-8, Kazusakamatari, Kisarazushi, Chiba-ken, 292-0818, Japan). Further, the JCM20101 strain can be obtained from the independent administrative institution, Institute of Physical and Chemical Research, Japan Collection of Microorganisms (JCM) (2-1, Hirosawa, Wako-shi, Saitama-ken, 351-0198, Japan). The ATCC 9625 strain can be obtained from American Type Culture Collection (Address: 12301 Parklawn Drive, Rockville, Maryland 20852, United States of America).

Then, the seed culture of each of the aforementioned strains was inoculated into a lactic medium having the same composition as mentioned above (sterilized at 95°C for 30 minutes) in an amount of 5.0 x 10⁶ to 2.0 x 10⁸ CFU per ml of the medium, and culture was performed at 37°C for 16 hours. The obtained culture broth was rapidly cooled to 10°C, and coagulation state, pH, and number of contained lactic acid bacteria were observed or measured. The number of lactic acid bacteria was measured on a commercially available BCP-added plate count agar plate (produced by EIKEN CHEMICAL CO., LTD.). The results are shown in Table 1. In the table, "E+N" means "x 10ⁿ".

**Table 1**

| Strain | Strain | PrtP | Cell count (CFU/g) | pH | Coagulation |
|---|---|---|---|---|---|
| *Lactococcus lactis* subsp. *lactis* | JCM20101 | + | 1.1E+09 | 4.65 | + |
| | NBRC 12007 | - | 3.0E+07 | 5.92 | - |
| | JCM 20128 | - | 4.4E+07 | 5.81 | - |
| | LcL 13 | - | 3.1E+08 | 5.75 | - |
| | LcL 26 | - | 1.8E+08 | 5.72 | - |
| | LcL 49 | - | 1.0E+08 | 5.59 | - |
| *Lactococcus lactis* subsp. *cremoris* | NBRC 100676 | + | 3.5E+08 | 4.78 | + |
| | ATCC 9625 | - | 4.2E+07 | 5.66 | - |
| | LcC 46 | - | 2.8E+08 | 5.41 | - |
| | LcC 53 | - | 3.1E+08 | 5.48 | - |

The *Lactococcus lactis* subspecies strains not having PrtP did not coagulate the lactic medium, as described in Yonezawa S. et al., J. Dairy Sci., 2010, 93:1815-23.

### Test Example 3: Mixed culture test with Lactococcus lactis not having PrtP and Bifidobacterium longum

First, the *Bifidobacterium longum* ATCC BAA-999 strain was inoculated into a medium containing 0.6% (W/W) of yeast extract and 11% (W/W) of reduced skim milk powder in an amount of 1.0 x 10⁶ to 5.0 x 10⁷ CFU per 1 ml of the medium, and cultured at 37°C for 16 hours to obtain seed culture.

Further, the culture medium of each strain of *Lactococcus lactis* obtained in Test Example 1 was inoculated in an amount of 3% into the Difco (registered trademark) M17 Broth (produced by Becton, Dickinson and Company) containing 0.5% each of lactose and glucose, and culture was performed at 30°C for 16 hours. The cultured cells of each strain were collected by centrifugation, washed, and suspended in a lactic medium having the same composition as mentioned above in the same volume as that of the original medium to obtain seed culture.

To a lactic medium having the same composition as described above (sterilized at 90°C for 10 minutes), the seed cultures of each strain of *Lactococcus lactis* and the *Bifidobacterium longum* ATCC BAA-999 strain were inoculated in amounts of 5.0 x 10⁶ to 2.0 x 10⁸ CFU and 1.0 x 10⁷ to 5.0 x 10⁷ CFU per 1 ml of the medium, respectively, and culture was performed at 37°C for 16 hours to obtain fermented milk.

The obtained fermented milk was rapidly cooled to 10°C, and pH, number of contained bifidobacteria, and dissolved oxygen were measured. The number of bifidobacteria was measured on a TOS propionate agar medium (produced by Yakult Pharmaceutical Industry Co., Ltd.) plate. Further, the dissolved oxygen was measured by using a fluorescence oxygen analyzer FO-960S (produced by ASR) with keeping the temperature of the fermented milk to be 10°C. The measurement results are shown in Table 2.

**Table 2**

| Strain | Strain | PrtP | Number of bifidobacteria (CFU/g) | | Survival rate of bifidobacteria (%) | pH | Dissolved oxygen concentration (ppm) | |
|---|---|---|---|---|---|---|---|---|
| | | | Immediately after the end of culture | After two weeks | | Immediately after the end of culture | Immediately after the end of culture | After two weeks |
| *Lactococcus lactis* subsp. *lactis* | JCM20101 | + | 2.4E+08 | 1.3E+08 | 54.2 | 4.93 | 0.5 or lower | 1.67 |
| | NBRC 12007 | - | 7.0E+06 | ND | - | 5.59 | 0.5 or lower | 5.53 |
| | JCM 20128 | - | 1.7E+07 | ND | - | 5.57 | 0.5 or lower | 6.68 |
| | LcL 13 | - | 5.30E+07 | 2.90E+07 | 54.7 | 4.90 | 0.5 or lower | 0.5 or lower |
| | LcL 26 | - | 1.43E+08 | 9.80E+07 | 68.7 | 4.86 | 0.5 or lower | 0.5 or lower |
| | LcL 49 | - | 1.8E+08 | 1.4E+08 | 77.6 | 5.00 | 0.5 or lower | 0.87 |
| *Lactococcus lactis* subsp. *cremoris* | NBRC 100676 | + | 2.8E+08 | 1.8E+08 | 64.3 | 4.98 | 0.5 or lower | 1.88 |
| | ATCC 9625 | - | 1.5E+07 | ND | - | 5.59 | 0.5 or lower | 6.80 |
| | FERM BP-6224 | - | 6.3E+07 | 8.4E+06 | 13.3 | 5.12 | 0.5 or lower | 5.24 |
| | LcC 46 | - | 1.28E+08 | 1.14E+08 | 89.0 | 4.88 | 0.5 or lower | 1.27 |
| | LcC 53 | - | 4.93E+07 | 4.70E+07 | 95.3 | 4.98 | 0.5 or lower | 1.77 |

| | | | | | | | | |
|---|---|---|---|---|---|---|---|---|
| ND: Lower than detection limit | | | | | | | | |

There were strains not having PrtP, but allowing survival of the bifidobacteria, like the strains having PrtP (JCM20101, NBRC 100676). The dissolved oxygen concentrations in the fermented milk observed with these strains after storage of two weeks were 2.0 ppm or lower.

### Test Example 4

Since most of the fermented milks containing bifidobacteria obtained in Test Example 3 showed pH around 5, they were in the form of drinkable yogurt. In order to produce fermented milk having a stronger structure (ordinary yogurt which can be taken in with a spoon), a lactic medium was fermented in the same manner as that of Test Example 3, but *Streptococcus thermophilus* and *Lactobacillus delbrueckii* subsp. *bulgaricus* were also added to the medium.

As in Test Example 3, seed cultures of each strain of *Lactococcus lactis* and the *Bifidobacterium longum* ATCC BAA-999 strain were inoculated to the lactic medium containing 1% (W/W) of glucose and 10% (W/W) of reduced skim milk powder in the same inoculation amounts as those used in Test Example 3, a yogurt starter containing *Streptococcus thermophilus* and *Lactobacillus delbrueckii* subsp. *bulgaricus* (produced by Danisco) was further added to the lactic medium in such an amount that the amounts of *Streptococcus thermophilus* and *Lactobacillus delbrueckii* subsp. *bulgaricus* were 2.0 x 10⁶ CFU and 2.0 x 10⁵ CFU per 1 ml of the medium, respectively, and culture was performed at 37°C for 8 hours to obtain fermented milk. The fermented milk was rapidly cooled to 10°C, and pH and coagulation state were measured or observed. The results are shown in Table 3.

**Table 3**

| Strain | Strain | PrtP | pH | Coagulation |
|---|---|---|---|---|
| Without *Lactococcus* bacterium | Without *Lactococcus* bacterium | | 4.87 | + |
| *Lactococcus lactis* subsp. *lactis* | JCM20101 | + | 4.87 | + |
| | LcL 13 | - | 4.86 | + |
| | LcL 26 | - | 4.85 | + |
| | LcL 49 | - | 5.47 | - |
| *Lactococcus lactis* subsp. *cremoris* | NBRC 100676T | + | 4.88 | + |
| | LcC 46 | - | 4.84 | + |
| | LcC 53 | - | 5.58 | - |

Although ordinary fermented milk could be produced in the system not containing *Lactococcus lactis* and the systems containing a strain having PrtP, pH was not decreased in the fermented milks produced with *Lactococcus lactis* subsp. *lactis* LcL49 and *Lactococcus lactis* subsp. *cremoris* LcC53, and they were not coagulated.

### Test Example 5: Evaluation of fermented milk

Fermented milk (yogurt) was produced by the method described in Example 3 described below, and evaluated for umami taste and bitterness in 10 grades by five panelists. A higher score means stronger tastes. A score exceeding 5 indicates that the fermented milk had unduly strong taste as fermented milk. The results are shown in Fig. 1.

As the results, strong umami taste and bitterness were sensed for the fermented milk produced by using a strain having PrtP, but for the fermented milk produced by using a strain not having PrtP, umami taste and bitterness were scarcely sensed, and the original flavor of fermented milk was not impaired.

### Example 1: Production of drinkable yogurt using Lactococcus lactis subsp. lactis

Into 1000 mL of a medium containing 10% (W/W) of reduced skim milk powder (sterilized at 90°C for 30 minutes), 30 mL of seed culture of the *Lactococcus lactis* subsp. *lactis* LcL26 strain was inoculated, and culture was performed at 25°C for 16 hours. Further, into 1000 mL of a medium containing 0.2% (W/W) of yeast extract and 11% (W/W) of reduced skim milk powder (sterilized at 90°C for 30 minutes), 100 mL of seed culture of the *Bifidobacterium longum* ATCC BAA-999 strain was inoculated, and culture was performed at 37°C for 4 hours.

The seed culture of the *Bifidobacterium longum* ATCC BAA-999 strain was obtained by inoculating 1.0 x 10⁶ to 5.0 x 10⁷ CFU of the *Bifidobacterium longum* ATCC BAA-999 strain into a medium containing 0.6% (W/W) of yeast extract and 11% (W/W) of reduced skim milk powder, and performing culture at 37°C for 16 hours.

Separately, skim milk powder, dry whole milk, sucrose and pectin as raw materials were mixed and dissolved to prepare 50 L of a milk raw material containing 0.5% (W/W) of milk fat, 8.0% (W/W) of fat-free milk solid content, 8.0% (W/W) of sucrose, and 0.2% (W/W) of pectin, and the obtained milk raw material was sterilized at 90°C for 10 minutes, and cooled to 40°C. Into this sterilized milk raw material, 500 mL of culture of the *Lactococcus lactis* subsp. *lactis* LcL26 strain and 500 mL of culture of the *Bifidobacterium longum* ATCC BAA-999 strain, which were precultured as described above, were inoculated, and culture was performed at 37°C for 16 hours to obtain fermented milk.

The obtained fermented milk was homogenized under a pressure of 15 MPa, filled in a 200-mL volume glass container, cooled until the temperature of the fermented milk became 10°C, and sealed to obtain drinkable yogurt. The obtained drinkable yogurt showed a lactic acid acidity of 0.64% and pH of 4.9, and contained 1.6 x 10⁸ CFU/ml of bifidobacteria. After storage at 10°C for 14 days, this drinkable yogurt contained 1.1 x 10⁸ CFU/ml of bifidobacteria, and the survival rate thereof was 68%. Further, dissolved oxygen concentration at this point was 0.93 ppm.

### Example 2: Production of drinkable yogurt using Lactococcus lactis subsp. cremoris

Into 1000 mL of a medium containing 10% (W/W) of reduced skim milk powder (sterilized at 90°C for 30 minutes), 30 mL of seed culture of the *Lactococcus lactis* subsp. *cremoris* LcC46 strain was inoculated, and culture was performed at 25°C for 16 hours. Further, into 1000 mL of a medium containing 0.2% (W/W) of yeast extract and 11% (W/W) of reduced skim milk powder (sterilizes at 90°C for 30 minutes), 100 mL of seed culture of the *Bifidobacterium longum* ATCC BAA-999 strain was inoculated, and culture was performed at 37°C for 4 hours.

The seed culture of the *Bifidobacterium longum* ATCC BAA-999 strain was obtained by inoculating 1.0 x 10⁶ to 5.0 x 10⁷ CFU of the *Bifidobacterium longum* ATCC BAA-999 strain into a medium containing 0.6% (W/W) of yeast extract and 11% (W/W) of reduced skim milk powder, and performing culture at 37°C for 16 hours.

Separately, skim milk powder, dry whole milk, sucrose and pectin as raw materials were mixed and dissolved to prepare 50 L of a milk raw material containing 0.5% (W/W) of milk fat, 8.0% (W/W) of fat-free milk solid content, 8.0% (W/W) of sucrose, and 0.2% (W/W) of pectin, and the obtained milk raw material was sterilized at 90°C for 10 minutes, and cooled to 40°C. Into this sterilized milk raw material, 500 mL of culture of the *Lactococcus lactis* subsp. *cremoris* LcC46 strain and 500 mL of culture of the *Bifidobacterium longum* ATCC BAA-999 strain, which were precultured as described above, were inoculated, and culture was performed at 37°C for 16 hours to obtain fermented milk.

The obtained fermented milk was homogenized under a pressure of 15 MPa, filled in a 200-mL volume glass container, cooled until the temperature of the fermented milk became 10°C, and sealed to obtain drinkable yogurt. The obtained drinkable yogurt showed a lactic acid acidity of 0.66% and pH of 4.8, and contained 9.6 x 10⁷ CFU/ml of bifidobacteria. After storage at 10°C for 14 days, this drinkable yogurt contained 6.9 x 10⁷ CFU/ml of bifidobacteria, and the survival rate thereof was 71%. Further, dissolved oxygen concentration at this point was 0.88 ppm.

### Example 3: Production of yogurt using Lactococcus lactis subsp. lactis (I)

Into 1000 mL of a medium containing 10% (W/W) of reduced skim milk powder (sterilized at 115°C for 20 minutes), 30 mL of seed culture of the *Lactobacillus delbrueckii* subsp. *lactis* FERM BP-10758 strain was inoculated, and culture was performed at 37°C for 16 hours. Further, into 1000 mL of a medium containing 0.1% (W/W) of yeast extract and 10% (W/W) of reduced skim milk powder (sterilized at 90°C for 30 minutes), 30 mL of seed culture of the *Streptococcus thermophilus* FERM P-17216 strain was inoculated, and culture was performed at 37°C for 5 hours.

The seed culture of the *Lactobacillus delbrueckii* subsp. *lactis* FERM BP-10758 strain was obtained by inoculating 1.0 x 10⁵ to 1.0 x 10⁷ CFU of the strain into a medium containing 0.1% (W/W) of yeast extract and 10% (W/W) of reduced skim milk powder, and performing culture at 37°C for 16 hours.

The seed culture of the *Streptococcus thermophilus* FERM P-17216 strain was obtained by inoculating 1.0 x 10⁵ to 1.0 x 10⁷ CFU of the strain into a medium containing 0.1% (W/W) of yeast extract and 10% (W/W) of reduced skim milk powder, and performing culture at 37°C for 16 hours.

Raw materials consisting of skim milk powder, cream, milk proteins and so forth were mixed and dissolved to prepare 50 L of a milk raw material containing 3.0% (W/W) of milk fat and 12.0% (W/W) of fat-free milk solid content, and the obtained milk raw material was warmed to 70°C, homogenized at a pressure of 15 MPa, sterilized at 90°C for 10 minutes, and cooled to 40°C.

Into this sterilized milk raw material, there were inoculated 50 mL of culture of the *Lactobacillus delbrueckii* subsp. *lactis* FERM BP-10758 strain and 450 mL of culture of the *Streptococcus thermophilus* FERM P-17216 strain, which were precultured as described above, as well as 500 mL of culture of the *Lactococcus lactis* subsp. *lactis* LcL13 strain and 500 mL of culture of the *Bifidobacterium longum* ATCC BAA-999 strain, which were obtained by preculture performed in the same manner as that used for the LcL26 strain in Example 1, and culture was performed at 37°C for 4 hours to obtain fermented milk. The obtained fermented milk was immediately stirred and cooled until the temperature of the fermented milk became 10°C, then filled into a 100-mL volume paper cup container, and sealed to obtain yogurt.

The obtained yogurt showed a lactic acid acidity of 0.74% and pH of 4.69, and contained 1.0 x 10⁸ CFU/ml of bifidobacteria. After storage at 10°C for 14 days, this yogurt contained 9.3 x 10⁷ CFU/ml of bifidobacteria, and the survival rate thereof was 93%. Further, dissolved oxygen concentration at this point was not higher than 0.5 ppm.

### Example 4: Production of yogurt using Lactococcus lactis subsp. lactis (II)

Skim milk powder, cream, and milk proteins were mixed and dissolved to prepare 50 L of a milk raw material containing 3.0% (W/W) of milk fat and 12.0% (W/W) of fat-free milk solid content, and the obtained milk raw material was warmed to 70°C, homogenized at a pressure of 15 MPa, sterilized at 90°C for 10 minutes, and cooled to 40°C.

Into this sterilized milk raw material, there were inoculated 500 mL of culture of the *Lactococcus lactis* subsp. *lactis* LcL26 strain and 500 mL of culture of the *Bifidobacterium longum* ATCC BAA-999 strain, which were obtained in the same manner as that of Example 1, as well as 0.002% of a yogurt starter containing *Streptococcus thermophilus* and *Lactobacillus delbrueckii* subsp. *bulgaricus* (produced by Danisco), and culture was performed at 37°C for 8 hours to obtain fermented milk. The obtained fermented milk was immediately stirred and cooled until the temperature of the fermented milk became 10°C, then filled into a 100-mL volume paper cup container, and sealed to obtain yogurt.

The obtained yogurt showed a lactic acid acidity of 0.65% and pH of 4.84, and contained 1.2 x 10⁸ CFU/ml of bifidobacteria. After storage at 10°C for 14 days, this yogurt contained 1.0 x 10⁸ CFU/ml of bifidobacteria, and the survival rate thereof was 83%. Further, dissolved oxygen concentration at this point was not higher than 0.5 ppm.

### Example 5: Production of yogurt using Lactococcus lactis subsp. lactis (III)

Skim milk powder, cream, and milk proteins were mixed and dissolved to prepare 50 L of a milk raw material containing 3.0% (W/W) of milk fat and 12.0% (W/W) of fat-free milk solid content, and the obtained milk raw material was warmed to 70°C, homogenized at a pressure of 15 MPa, sterilized at 90°C for 10 minutes, and cooled to 40°C.

Into this sterilized milk raw material, there were inoculated 500 mL of culture of the *Lactococcus lactis* subsp. *lactis* LcL26 strain obtained in the same manner as that of Example 1, 1.5 x 10¹⁴ CFU (colony forming unit) of frozen cells of the *Bifidobacterium longum* ATCC BAA-999 strain (produced by Morinaga Milk Industry Co., Ltd.), and 0.002% of a yogurt starter containing *Streptococcus thermophilus* and *Lactobacillus delbrueckii* subsp. *bulgaricus* (produced by Danisco), and culture was performed at 37°C for 4 hours to obtain fermented milk. The obtained fermented milk was immediately stirred and cooled until the temperature of the fermented milk became 10°C, then filled into a 100-mL volume paper cup container, and sealed to obtain yogurt.

The obtained yogurt showed a lactic acid acidity of 0.70% and pH of 4.74, and contained 4.2 x 10⁹ CFU/ml of bifidobacteria. After storage at 10°C for 14 days, this yogurt contained 2.0 x 10⁹ CFU/ml of bifidobacteria, and the survival rate thereof was 47.6%. Further, dissolved oxygen concentration at this point was 1.47 ppm.

### Industrial Applicability

According to the method for producing a fermented food of the present invention, a fermented food containing a large amount of *Bifidobacterium* bacteria, especially *Bifidobacterium longum,* can be efficiently produced. Further, the fermented food produced by the method for producing a fermented food of the present invention is of course useful for health care, and is a preferred fermented food showing superior flavor.

Further, the starter for fermentation of a milk raw material containing a *Bifidobacterium* bacterium of the present invention can be used for production of the fermented food.

## Claims

1. A method for producing a fermented food, which comprises fermenting a milk raw material by using *Lactococcus lactis* not having cell wall-enveloped proteinase and a *Bifidobacterium* bacterium.

2. The method according to claim 1, wherein the *Lactococcus lactis* has a property that, when said bacterium is inoculated into a medium containing 1% (W/W) of glucose and 10% (W/W) of reduced skim milk powder in an amount of 5.0 x 10⁶ to 2.0 x 10⁸ CFU per 1 ml of the medium, and cultured at 37°C for 4 to 24 hours, the medium is not coagulated.

3. The method according to claim 1 or 2, wherein the *Lactococcus lactis* has a property that, when said bacterium and the *Bifidobacterium* bacterium are inoculated into a medium containing 1% (W/W) of glucose and 10% (W/W) of reduced skim milk powder in amounts of 5.0 x 10⁶ to 2.0 x 10⁸ CFU and 1.0 x 10⁷ to 3.0 x 10⁹ CFU per 1 ml of the medium, respectively, and cultured, and the medium is rapidly cooled to 10°C from culture temperature when pH of the medium becomes 4.6 to 5.5, and stored at 10°C for 2 weeks, dissolved oxygen concentration in the medium is maintained to be 2 ppm or lower.

4. The method according to any one of claims 1 to 3, wherein the *Lactococcus lactis* has a property that, when said bacterium and the *Bifidobacterium* bacterium are inoculated into a medium containing 1% (W/W) of glucose and 10% (W/W) of reduced skim milk powder in amounts of 5.0 x 10⁶ to 2.0 x 10⁸ CFU and 1.0 x 10⁷ to 3.0 x 10⁹ CFU per 1 ml of the medium, respectively, and cultured, and the medium is rapidly cooled to 10°C from culture temperature when pH of the medium becomes 4.6 to 5.5, and stored at 10°C for 2 weeks, survival rate of the *Bifidobacterium* bacterium is maintained to be 30% or more.

5. The method according to any one of claims 1 to 4, wherein the *Lactococcus lactis* has a property that, when said bacterium, the *Bifidobacterium* bacterium, *Streptococcus thermophilus* and *Lactobacillus delbrueckii* subsp. *bulgaricus* are inoculated into a medium containing 1% (W/W) of glucose and 10% (W/W) of reduced skim milk powder in amounts of 5.0 x 10⁶ to 2.0 x 10⁸ CFU, 1.0 x 10⁷ to 3.0 x 10⁹ CFU, 1.0 x 10³ to 9.0 x 10⁷ CFU and 1.0 x 10³ to 9.0 x 10⁷ CFU per 1 ml of the medium, respectively, and cultured at 37°C for 3 to 24 hours, the medium is coagulated.

6. The method according to any one of claims 1 to 5, wherein the *Lactococcus lactis* is selected from the group consisting of *Lactococcus lactis* subsp. *lactis* and *Lactococcus lactis* subsp. *cremoris.*

7. The method according to any one of claims 1 to 6, wherein the *Lactococcus lactis* is selected from the group consisting of *Lactococcus lactis* subsp. *lactis* LcL13 (FERM BP-11276), *Lactococcus lactis* subsp. *lactis* LcL26 (FERM BP-11277), and *Lactococcus lactis* subsp. *cremoris* LcC46 (FERM BP-11275).

8. The method according to any one of claims 1 to 7, wherein the *Bifidobacterium* bacterium is *Bifidobacterium longum.*

9. The method according to claim 8, wherein the *Bifidobacterium longum* is the *Bifidobacterium longum* ATCC BAA-999 strain.

10. The method according to any one of claims 1 to 9, wherein a lactic acid bacterium selected from the group consisting of *Streptococcus thermophilus* and *Lactobacillus delbrueckii* is further used for the fermentation.

11. A fermented food produced by the method according to any one of claims 1 to 10.

12. A starter for fermentation of a milk raw material containing a *Bifidobacterium* bacterium, which comprises *Lactococcus lactis* not having cell wall-enveloped proteinase.

13. The starter for fermentation of a milk raw material comprising a *Bifidobacterium* bacterium according to claim 12, wherein the *Lactococcus lactis* has a property that, when said bacterium is inoculated into a medium containing 1% (W/W) of glucose and 10% (W/W) of reduced skim milk powder at 5.0 x 10⁶ to 2.0 x 10⁸ CFU per 1 ml of the medium, and cultured at 37°C for 4 to 24 hours, the medium is not coagulated.

14. The starter for fermentation of a milk raw material comprising a *Bifidobacterium* bacterium according to claim 12 or 13, wherein the *Lactococcus lactis* has a property that, when said bacterium and the *Bifidobacterium* bacterium are inoculated into a medium containing 1% (W/W) of glucose and 10% (W/W) of reduced skim milk powder in amounts of 5.0 x 10⁶ to 2.0 x 10⁸ CFU and 1.0 x 10⁷ to 3.0 x 10⁹ CFU per 1 ml of the medium, respectively, and cultured, and the medium is rapidly cooled to 10°C from culture temperature when pH of the medium becomes 4.6 to 5.5, and stored at 10°C for 2 weeks, survival rate of the *Bifidobacterium* bacterium is maintained to be 30% or more.

15. The starter for fermentation of a milk raw material comprising a *Bifidobacterium* bacterium according to any one of claims 12 to 14, wherein the *Lactococcus lactis* has a property that, when said bacterium, the *Bifidobacterium* bacterium, *Streptococcus thermophilus* and *Lactobacillus delbrueckii* subsp. *bulgaricus* are inoculated into a medium containing 1% (W/W) of glucose and 10% (W/W) of reduced skim milk powder in amounts of 5.0 x 10⁶ to 2.0 x 10⁸ CFU, 1.0 x 10⁷ to 3.0 x 10⁹ CFU, 2.0 x 10⁵ to 9.0 x 10⁷ CFU and 2.0 x 10⁵ to 9.0 x 10⁷ CFU per 1 ml of the medium, respectively, and cultured at 37°C for 3 to 24 hours, the medium is coagulated.

16. The starter for fermentation of a milk raw material comprising a *Bifidobacterium* bacterium according to any one of claims 12 to 15, wherein the *Lactococcus lactis* is selected from the group consisting of *Lactococcus lactis* subsp. *lactis* and *Lactococcus lactis* subsp. *cremoris.*

17. The starter for fermentation of a milk raw material comprising a *Bifidobacterium* bacterium according to any one of claims 12 to 16, wherein the *Lactococcus lactis* is selected from the group consisting of *Lactococcus lactis* subsp. *lactis* LcL13 (FERM BP-11276), *Lactococcus lactis* subsp. *lactis* LcL26 (FERM BP-11277), and *Lactococcus lactis* subsp. *cremoris* LcC46 (FERM BP-11275).

18. The starter for fermentation of a milk raw material comprising a *Bifidobacterium* bacterium according to any one of claims 12 to 17, wherein the *Bifidobacterium* bacterium is *Bifidobacterium longum.*

19. The starter for fermentation of a milk raw material comprising a *Bifidobacterium* bacterium according to claim 18, wherein the *Bifidobacterium longum* is the *Bifidobacterium longum* ATCC BAA-999 strain.

20. A bacterial strain selected from the group consisting of *Lactococcus lactis* subsp. *lactis* LcL13 (FERM BP-11276), *Lactococcus lactis* subsp. *lactis* LcL26 (FERM BP-11277), and *Lactococcus lactis* subsp. *cremoris* LcC46 (FERM BP-11275).
